# EUROPEAN PATENT SPECIFICATION

(11) **EP 1 498 118 B1**
(45) Date of publication and mention of the grant of the patent: **12.09.2007**
(21) Application number: 03717562.7
(22) Date of filing: 11.04.2003
(51) Int. Cl.: A61K 31/137, A61K 47/10, A61K 47/14, A61K 9/70, A61P 9/02

(54) **PATCH AND PROCESS FOR PRODUCING THE SAME**
PFLASTER UND VERFAHREN ZU SEINER HERSTELLUNG
TIMBRE TRANSDERMIQUE ET PROCEDE DE PRODUCTION ASSOCIE

(30) Priority: 12.04.2002 JP 2002110609
(43) Date of publication of application: 19.01.2005
(73) Proprietor: Meiji Dairies Corporation, Koto-ku, Tokyo 136-8908 (JP)
(72) Inventor: HORI, Mitsuhiko c/o Nitto Denko Corporation, Ibaraki-shi, Osaka 567-8680 (JP); YAMAMOTO, Keiji c/o Nitto Denko Corporation, Ibaraki-shi, Osaka 567-8680 (JP); MATSUOKA, Kensuke c/o Nitto Denko Corporation, Ibaraki-shi, Osaka 567-8680 (JP); NAKANO, Yoshihisa c/o Nitto Denko Corporation, Ibaraki-shi, Osaka 567-8680 (JP); MINOMI, Kenjirou c/o Nitto Denko Corporation, Ibaraki-shi, Osaka 567-8680 (JP); YAMADA, Masashi c/o Pharmaceuticals Department, Koto-ku, Tokyo 136-8908 (JP); OHTSUKA, Masaru, Sagamihara-shi, Kanagawa 229-0002 (JP); SUZUKI, Yasunori c/o Pharmaceuticals Department, Odawara-shi, Kanagawa 250-0862 (JP); KAWASHIMA, Akihiro c/o Pharmaceuticals Department, Odawara-shi, Kanagawa 250-0862 (JP)
(74) Representative: Weber, Thomas
(86) International application number: PCT/JP2003/004606
(87) International publication number: WO 2003/086376

(56) References cited:
- WO-A-01/89473
- WO-A1-01/89473
- JP-A- 1 085 911
- JP-A- 3 112 556
- JP-A- 9 143 062
- JP-A- 11 139 968
- JP-A- 11 512 080
- JP-A- 57 007 413
- JP-A- 57 007 414
- JP-A- 59 172 418
- JP-A- 60 016 922
- JP-A- 2001 131 062
- JP-A- 2001 512 465
- US-A- 5 215 751
- US-A- 5 462 746
- US-B1- 6 348 210
- PATENT ABSTRACTS OF JAPAN vol. 1999, no. 10, 31 August 1999 (1999-08-31) & JP 11 139968 A (TOA EIYOO KK), 25 May 1999 (1999-05-25)

## Description

### Technical Field

The present invention relates to a patch for percutaneous administration of 2-amino-1-(2',5'-dimethoxyphenyl)ethanol (hereinafter to be referred to as DMAE) or a pharmacologically acceptable salt thereof (hereinafter to be generally referred to as DMAEs), and a production method thereof.

### Background Art

DMAE is an active metabolite of Midodrine hydrochloride, which is a therapeutic agent for selective α₁-receptor stimulating hypotension. Midodrine hydrochloride is used for the treatment of essential hypotension and orthostatic hypotension, and further expected to be applicable to the treatment of stress urinary incontinence utilizing its smooth muscle contracting action. The signature and dose of Midodrine hydrochloride is generally two times of administration of a 2 mg tablet per day. In the case of oral administration, a drug taken into the body cannot avoid decomposition by the digestive tract and primary metabolism in the liver. Assuming the application to stress urinary incontinence, moreover, patients are mostly aged, and administration is difficult to confirm due to missed dose and the like. In consideration of availability of the administered drug, retention of pharmacological effect, convenience of administration, compliance such as confirmation of administration and the like, therefore, a method for percutaneous administration through the skin, particularly a drug administration method comprising use of a patch for adhesion of a drug-containing adhesive layer to the skin is desirably employed.

However, since percutaneous absorbability of Midodrine hydrochloride and Midodrine is extremely low, percutaneous administration of its active metabolite, DMAE, is desired. Nevertheless, percutaneous absorbability of DMAE itself is also insufficient to express an expected pharmacological effect, and for a sufficient pharmacological effect to be expressed, an absorption promoter represented by an organic liquid component (e.g., long chain fatty acid ester, long chain aliphatic alcohol etc.) needs to be added to an adhesive layer of a patch.

The addition of an organic liquid component to an adhesive layer is extremely useful for improving percutaneous absorbability of DMAE contained in the adhesive layer. However, when an organic liquid component is added in a large amount, the adhesive is excessively plasticized reducing its cohesive power, which then causes problems in that the adhesive partially remains on the skin upon peeling off of the patch from the skin after adhesion (i.e., adhesive residue), and a part of the adhesive leaks out from the edge of the adhesive layer during storage of a patch in a package (i.e., adhesive bleed) and adheres to the inside of the package, thereby preventing the patch from being taken out easily.

To prevent a decrease of the cohesive power of an adhesive, the adhesive is generally crosslinked using various crosslinking agents such as isocyanate, metal salts (metal chelate compound), epoxy and the like. It has been also found that problems exist in that, when DMAE is contained in an adhesive, these crosslinking agents cannot be used, because adhesives and DMAE react to inhibit crosslinking of the adhesive during preparation of an adhesive layer, and these crosslinking agents disturb stability of DMAE during preparation of the adhesive layer.

WO 01 89473 A1 discloses a transdermal delivery system of desglymidodrine comprising a backing layer, an adhesive layer of polyacrylate, an active layer of an PVP-hydrogel and a liner.

US-B1-6 348 210 discloses a transdermal patch comprising a non-crosslinked drug adhesive layer.

Accordingly, it is an object of the present invention to provide a patch that avoids problems such as adhesive residue and adhesive bleed, that facilitates addition of a percutaneous absorption agent, and that improves the percutaneous absorbability of DMAEs, as well as a production method thereof.

### Disclosure of the Invention

As a result of the intensive studies made by the present inventors in an attempt to solve the above-mentioned problems, it has been found that a patch comprising a substrate, a non-crosslinked adhesive layer containing DMAEs (to be referred to as an adhesive layer (A) in the present specification), which is laminated on one surface of the substrate, and a crosslinked adhesive layer (to be referred to as a crosslinked adhesive layer (B) in the present specification) laminated on the adhesive layer (A), improves percutaneous absorbability of DMAEs and is free of the problems of adhesive residue and adhesive bleed, which resulted in the completion of the present invention.

Accordingly, the present invention provides the following.
[1] A patch comprising a substrate, a non-crosslinked adhesive layer (A) comprising 2-amino-1-(2',5'-dimethoxyphenyl)ethanol or a pharmacologically acceptable salt thereof, which is laminated on one surface of the substrate, and a crosslinked adhesive layer (B) laminated on the adhesive layer (A).
[2] The patch of the above-mentioned [1], wherein the crosslinked adhesive layer (B) is obtained by crosslinking an adhesive with at least one kind of crosslinking agent selected from the group consisting of an isocyanate crosslinking agent, a metal salt crosslinking agent and an epoxy crosslinking agent.
[3] The patch of the above-mentioned [1], wherein the adhesive layer (A) and/or the crosslinked adhesive layer (B) comprise(s) an acrylic adhesive.
[4] The patch of the above-mentioned [1], wherein the adhesive layer (A) and/or the crosslinked adhesive layer (B) comprise(s) a long chain fatty acid ester and/or a long chain aliphatic alcohol.
[5] The patch of the above-mentioned [4], which satisfies at least one of the following (i) and (ii):
   (i) the total content of the long chain fatty acid ester and/or the long chain aliphatic alcohol in the adhesive layer (A) is 25-200 parts by weight per 100 parts by weight of the adhesive in the adhesive layer (A),
   (ii) the total content of the long chain fatty acid ester and/or the long chain aliphatic alcohol in the crosslinked adhesive layer (B) is 25-200 parts by weight per 100 parts by weight of the adhesive in the crosslinked adhesive layer (B).
[6] The patch of the above-mentioned [4], wherein the long chain fatty acid ester is an ester of a fatty acid having 8 to 30 carbon atoms and an alcohol having 1 to 18 carbon atoms and the long chain aliphatic alcohol has 8 to 30 carbon atoms.
[7] The patch of the above-mentioned [1], wherein the content of 2-amino-1-(2',5'-dimethoxyphenyl)ethanol or a pharmacologically acceptable salt thereof in the adhesive layer (A) is 0.5-60 wt% of the total weight of the adhesive layer (A).
[8] The patch of the above-mentioned [1], wherein the substrate is a laminate of a plastic film and a non-woven fabric and the adhesive layer (A) is laminated on the non-woven fabric side.
[9] The patch of the above-mentioned [1], wherein the adhesive in the adhesive layer (A) and the adhesive in the crosslinked adhesive layer (B) have the same composition.
[10] A production method of a patch, which comprises the steps of
   (1) dissolving a non-crosslinked adhesive and 2-amino-1-(2',5'-dimethoxyphenyl)ethanol or a pharmacologically acceptable salt thereof in a non-ester organic solvent to give an adhesive solution,
   (2) applying the adhesive solution onto one surface of a substrate, and drying the adhesive solution to form an adhesive layer (A), or applying the adhesive solution onto a separator, drying the adhesive solution to form an adhesive layer and transfer-coating the adhesive layer on one surface of a substrate to form an adhesive layer (A), and
   (3) forming a crosslinked adhesive layer (B) free of 2-amino-1-(2',5'-dimethoxyphenyl)ethanol and a pharmacologically acceptable salt thereof on the adhesive layer (A), in this order.
[11] The method of the above-mentioned [10], wherein the crosslinked adhesive layer (B) is obtained by crosslinking an adhesive with at least one kind of crosslinking agent selected from the group consisting of an isocyanate crosslinking agent, a metal salt crosslinking agent and an epoxy crosslinking agent.
[12] The method of the above-mentioned [10], wherein the non-ester organic solvent is at least one kind selected from the group consisting of toluene, hexane, methanol, ethanol and propanol.

### Detailed Description of the Invention

The present invention is explained in detail in the following.

The DMAEs contained in the adhesive layer (A) are mainly used with the hope for the treatment of essential hypotension and orthostatic hypotension, and of stress urinary incontinence utilizing its smooth muscle contracting action. Use of DMAEs is not limited to these and DMAEs may exhibit different pharmacological actions.

As the pharmacologically acceptable salts of DMAE, for example, salts with inorganic acid or organic acid can be mentioned. As the inorganic acid, for example, hydrochloric acid, hydrobromic acid, nitric acid, sulfuric acid, phosphoric acid and the like can be mentioned, and as the organic acid, formic acid, acetic acid, trifluoroacetic acid, propionic acid, lactic acid, tartaric acid, oxalic acid, fumaric acid, maleic acid, citric acid, malonic acid, methanesulfonic acid and the like can be mentioned.

As the adhesive to be used for the adhesive layer (A), a medical adhesive having tackiness at an ambient temperature, such as an acrylic adhesive, a natural rubber adhesive, a synthetic rubber adhesive (e.g., synthetic isoprene rubber, polyisobutyrene rubber, styrene/butadiene rubber, styrene/isoprene/styrene rubber, styrene/butadiene/styrene rubber and the like), a silicone adhesive, a vinyl ester adhesive, a vinyl ether adhesive and the like are preferable. Of these, at least one kind of adhesive selected from the group consisting of acrylic, natural rubber, synthetic rubber and a silicone adhesive is preferably used, which is particularly preferably an acrylic adhesive, from the aspects of the stable quality of an adhesive and easiness of control of the adhesive properties. The adhesive to be used for the adhesive layer (A) may be used alone or in combination with plural kinds of adhesives where necessary.

The adhesive layer (A) needs to be essentially non-crosslinked. The absence of a crosslinking agent for formation of the adhesive layer (A) contributes to the prevention of degraded stability of DMAEs due to the contact of the crosslinking agent with DMAEs.

The above-mentioned acrylic adhesive is not particularly limited and is exemplified by a (meth)acrylate adhesive, preferably a copolymer of an alkyl(meth)acrylate and a copolymerizable monomer to be mentioned below. For example, a copolymer obtained by copolymerization of 40-99 wt% of alkyl(meth)acrylate and 1-60 wt% of a copolymerizable monomer can be mentioned, with preference given to a copolymer obtained by copolymerization of 50-98 wt% of alkyl(meth)acrylate and 2-50 wt% of a copolymerizable monomer wherein the total weight of the copolymer is 100 wt%. The alkyl(meth)acrylate and the copolymerizable monomer can be respectively used in combination of one or more thereof.

As such alkyl (meth) acrylate, an ester obtained from primary-tertiary alcohol wherein the alkyl group has 2-18, preferably 4-12, carbon atoms and acrylic acid or methacrylic acid can be preferably used.

Specific examples thereof include ethyl(meth)acrylate, butyl(meth)acrylate, tert-butyl(meth)acrylate, pentyl(meth)acrylate, hexyl(meth)acrylate, heptyl(meth)acrylate, octyl(meth)acrylate, isooctyl(meth)acrylate, nonyl(meth)acrylate, isononyl(meth)acrylate, decyl(meth)acrylate, undecyl(meth)acrylate, dodecyl(meth)acrylate, 2-ethylhexyl(meth)acrylate and the like.

In contrast, as the copolymerizable monomer, a monomer having at least one unsaturated double bond in the molecule, which is involved in the copolymerization reaction, and a functional group in the side chain, such as carboxyl group (e.g., (meth)acrylic acid, itaconic acid, maleic acid, maleic anhydride and the like), hydroxyl group (e.g., hydroxyethyl(meth)acrylate, hydroxypropyl(meth)acrylate and the like), sulfoxyl group (e.g., styrene sulfonic acid, allylsulfonic acid, sulfopropyl(meth)acrylate, (meth)acryloyloxynaphthalenesulfonic acid, acrylamide methylpropanesulfonic acid and the like), amino group (e.g., aminoethyl(meth)acrylate, dimethylaminoethyl(meth)acrylate, tert-butylaminoethyl(meth)acrylate and the like), amido group (e.g., (meth)acrylamide, dimethyl(meth)acrylamide, N-butyl (meth) acrylamide, N-methylol(meth)acrylamide, N-methylolpropane(meth)acrylamide and the like), alkoxyl group (e.g., methoxyethyl(meth)acrylate (e.g., 2-methoxyethyl acrylate and the like), ethoxyethyl(meth)acrylate, methoxyethylene glycol(meth)acrylate, methoxydiethylene glycol(meth)acrylate, methoxytriethylene glycol(meth)acrylate, methoxypolyethylene glycol(meth)acrylate, tetrahydrofurfuryl(meth)acrylate and the like) and the like, can be used. As the copolymerizable monomer other than these, for example, (meth)acrylonitrile, methyl (meth) acrylate, and vinyl monomers such as vinyl acetate, vinyl propionate, vinylpyrrolidone (e.g., N-vinyl-2-pyrrolidone and the like), methylvinylpyrrolidone, vinylpyridine, vinylpiperidone, vinylpyrimidine, vinylpiperazine, vinylpyrazine, vinylpyrrole, vinylimidazole, vinyl caprolactam, vinyloxazole, vinylmorpholine and the like can be used.

As the copolymerizable monomer, a carboxyl group-containing monomer and/or a hydroxyl group-containing monomer is/are preferably used from among the above-exemplified monomers, in view of the adhesiveness and cohesiveness as the adhesive properties, releasability of DMAEs contained in the adhesive layer, and the like. They are preferably copolymerized in the range of generally 1-50 wt%, preferably 3-20 wt%. When a vinyl monomer is used, vinyl acetate and N-vinyl-2-pyrrolidone are preferably used and these are used in a proportion of generally not more than 40 wt%, preferably not more than 30 wt%.

As the acrylic adhesive, for example, a copolymer of 2-ethylhexyl acrylate and acrylic acid, a copolymer of 2-ethylhexyl acrylate and hydroxyethyl acrylate, a copolymer of 2-ethylhexyl acrylate and methyl methacrylate, a copolymer of 2-ethylhexyl acrylate, 2-methoxyethyl acrylate and vinyl acetate, a copolymer of 2-ethylhexyl acrylate and vinylpyrrolidone, a copolymer of 2-ethylhexyl acrylate, methyl methacrylate and 2-methoxyethyl acrylate, a copolymer of 2-ethylhexyl acrylate, vinylpyrrolidone and acrylic acid, and the like can be specifically mentioned.

The adhesive layer (A) may further contain rosin, rosin derivative, polyterpene resin, coumarone-indene resin, petroleum resin, terpene phenol resin and the like as necessary to increase viscosity.

The content of DMAEs in the adhesive layer (A) is in the range of generally 0.5-60 wt%, preferably 5-50 wt%, particularly preferably 15-40 wt%, of the total weight of the adhesive layer (A).

By setting the content of DMAEs for generally not less than 0.5 wt%, preferably not less than 5 wt%, particularly preferably not less than 15 wt%, of the total weight of the adhesive layer (A), a sufficient amount of the drug for showing a pharmacological effect can be percutaneously absorbed.

By setting the content of DMAEs for generally not more than 60 wt%, preferably not more than 50 wt%, particularly preferably not more than 40 wt%, of the total weight of the adhesive layer (A), degradation of the adhesiveness of the adhesive layer (A) can be prevented, and the adhesive layer (A) can be sufficiently adhered to the crosslinked adhesive layer (B).

The adhesive layer (A) can contain an organic liquid component. As the organic liquid component, for example, long chain fatty acid ester, long chain aliphatic alcohol and the like can be mentioned. By adding an organic liquid component such as long chain fatty acid ester, long chain aliphatic alcohol and the like, these components become compatible with the adhesive layer to plasticize the adhesive layer. As a result, the diffusability of DMAEs in the adhesive layer can be improved, the skin permeability can be promoted and the percutaneous absorbability of DMAEs can be improved. The organic liquid component such as long chain fatty acid ester, long chain aliphatic alcohol and the like can be used in combination of one or more kinds thereof.

As the long chain fatty acid ester, for example, an ester of a fatty acid having 8 to 30 carbon atoms and an alcohol having 1 to 18 carbon atoms can be mentioned. Specific examples include isopropyl myristate, diethyl sebacate, octyl palmitate, ethyl oleate, laurate (e.g., hexyl laurate and the like), fatty acid esters of glycerol (e.g., glycerol monomyristate, glycerol monostearate and the like), fatty acid esters of propylene glycol (e.g., propylene glycol monostearate and the like) and the like.

As the long chain aliphatic alcohol, for example, aliphatic alcohol having 8 to 30 carbon atoms can be mentioned. Specific examples thereof include octyl alcohol, decyl alcohol, dodecyl alcohol, oleyl alcohol, isostearyl alcohol, hexyl decanol, octyl dodecanol, lauryl alcohol and the like.

The total content of the organic liquid component in the adhesive layer (A) is in the range of generally 25-200 parts by weight, preferably 40-180 parts by weight, particularly preferably 50-150 parts by weight, per 100 parts by weight of the adhesive in the adhesive layer (A).

By setting the content of the organic liquid component in the adhesive layer (A) for generally not less than 25 parts by weight, preferably not less than 40 parts by weight, particularly preferably not less than 50 parts by weight, per 100 parts by weight of the adhesive in the adhesive layer (A), the adhesive layer can be sufficiently plasticized, as a result of which, diffusability of DMAEs in the adhesive layer can be improved to promote its skin permeability, which in turn results in an improved percutaneous absorbability of DMAEs.

By setting the content of the organic liquid component in the adhesive layer (A) for generally not more than 200 parts by weight, preferably not more than 180 parts by weight, particularly preferably not more than 150 parts by weight, per 100 parts by weight of the adhesive in the adhesive layer (A), a sufficient cohesive power can be maintained even without crosslinking.

As the adhesive to be used for the crosslinked adhesive layer (B), conventionally used medical adhesives such as acrylic adhesive, a natural rubber adhesive, a synthetic rubber adhesive (e.g., synthetic isoprene rubber, polyisobutyrene rubber, styrene/butadiene rubber, styrene/isoprene/styrene rubber, styrene/butadiene/styrene rubber and the like), a silicone adhesive, a vinyl ester adhesive, a vinyl ether adhesive and the like, which have tackiness at ambient temperature and which are free of rash and the like upon application to the skin surface, are preferable. Of these, at least one kind of adhesive selected from the group consisting of acrylic, natural rubber, synthetic rubber and a silicone adhesive, particularly preferable acrylic adhesive, is preferably used from the aspects of stable quality of adhesive and easy control of adhesive properties. The adhesive to be used for the crosslinked adhesive layer (B) may be used alone or in combination with plural kinds of adhesives where necessary.

The above-mentioned acrylic adhesive is not particularly limited and is exemplified by a (meth)acrylate adhesive, preferably a copolymer of an alkyl(meth)acrylate and a copolymerizable monomer to be mentioned below. For example, a copolymer obtained by copolymerization of 40-99 wt% of alkyl(meth)acrylate and 1-60 wt% of a copolymerizable monomer can be mentioned, with preference given to a copolymer obtained by copolymerization of 50-98 wt% of alkyl(meth)acrylate and 2-50 wt% of a copolymerizable monomer wherein the total weight of the copolymer is 100 wt%. The alkyl(meth)acrylate and the copolymerizable monomer may be respectively used in combination of one or more thereof.

As such alkyl(meth)acrylate and copolymerizable monomer, those exemplified for the aforementioned adhesive layer (A) can be preferably used.

The copolymerizable monomer in combination of one or more kinds thereof can be copolymerized with alkyl(meth)acrylate, as mentioned above. In view of the adhesive properties such as adhesiveness, cohesiveness and the like, a total amount in the range of generally 1-50 wt%, preferably 3-20 wt%, of at least one of the carboxyl group-containing monomer and the hydroxyl group-containing monomer is copolymerized, and where necessary, the above-exemplified other monomer; for example, vinyl monomer such as vinyl acetate, N-vinyl-2-pyrrolidone and the like, is preferably copolymerized in a proportion of generally not more than 40 wt%, preferably not more than 30 wt%.

As the acrylic adhesive, for example, a copolymer of 2-ethylhexyl acrylate and acrylic acid, a copolymer of 2-ethylhexyl acrylate and hydroxyethyl acrylate, a copolymer of 2-ethylhexyl acrylate, vinylpyrrolidone and acrylic acid, and the like can be specifically mentioned.

The crosslinking treatment of the adhesive is not particularly limited and can be conducted by, for example, a conventional method using a crosslinking agent. The crosslinking agent is not particularly limited, and for example, isocyanates (e.g., CORONATE HL: manufactured by NIPPON POLYURETHANE INDUSTRY CO., LTD., and the like), metal salts (metal chelate compounds) (e.g., ALCH: manufactured by Kawaken Fine Chemicals Co., Ltd., and the like), epoxy (e.g., TEPIC: manufactured by NISSAN CHEMICAL INDUSTRIES LTD., and the like) and the like can be used. The crosslinking agent may be used alone or in combination of plural kinds thereof where necessary.

While the content of the crosslinking agent varies depending on the kind of the crosslinking agent, it is in the range of generally 0.01-5 parts by weight, preferably 0.03-3 parts by weight, particularly preferably 0.05-1 part by weight, per 100 parts by weight of the adhesive to be crosslinked.

The adhesive in the adhesive layer (A) and the adhesive in the crosslinked adhesive layer (B) preferably have the same composition for the prevention of interfacial peeling between both adhesive layers upon adhesion of the adhesive layers, promotion of migration of DMAEs between the both adhesive layers, and improvement of adhesiveness of both adhesive layers. By the "same composition" is meant that the kind of the adhesives is the same. When plural kinds of adhesives are used, the kind and the content of the adhesives are the same.

The crosslinked adhesive layer (B) may further contain rosin, rosin derivative, polyterpene resin, coumarone-indene resin, petroleum resin and terpene phenol resin and the like as necessary to increase the viscosity.

When the crosslinked adhesive layer (B) is prepared by a crosslinking treatment, as mentioned below in the production process of the patch according to the present invention, the absence of DMAEs in the adhesive can avoid inhibition of crosslinking of the adhesive, which can be caused by the contact between the crosslinking agent and DMAEs. Furthermore, because the crosslinked adhesive layer (B) after completion of the crosslinking treatment is free of an unreacted crosslinking agent of a level that can affect the stability of DMAEs, the subsequent migration of DMAEs from the adhesive layer (A) does not pose any problem.

The crosslinked adhesive layer (B) may contain an organic liquid component. As the organic liquid component, for example, long chain fatty acid ester, long chain aliphatic alcohol and the like can be mentioned. By adding an organic liquid component such as long chain fatty acid ester, long chain aliphatic alcohol and the like, the skin permeability of DMAEs is promoted, and as a result, the percutaneous absorbability of DMAEs can be improved. In addition, these components have an effect of plasticizing the adhesive layer by being compatible with the adhesive layer. When the patch is adhered to the skin surface, it gives a soft feeling as well. Furthermore, by the above-mentioned crosslinking treatment of the adhesive, a suitable cohesive power is afforded to the adhesive, and irritation to the skin upon peeling off after use can be reduced as much as possible. The organic liquid component such as long chain fatty acid ester, long chain aliphatic alcohol and the like can be used in combination with one or more kinds thereof.

As the long chain fatty acid ester and long chain aliphatic alcohol, those exemplified for the aforementioned adhesive layer (A) can be preferably used.

The total content of the organic liquid component in the crosslinked adhesive layer (B) is in the range of generally 25-200 parts by weight, preferably 40-180 parts by weight, particularly preferably 50-150 parts by weight, per 100 parts by weight of the adhesive in the crosslinked adhesive layer (B).

By setting the content of the organic liquid component in the crosslinked adhesive layer (B) for generally not less than 25 parts by weight, preferably not less than 40 parts by weight, particularly preferably not less than 50 parts by weight, per 100 parts by weight of the adhesive in the crosslinked adhesive layer (B), the skin permeability of DMAEs can be promoted and sufficient plasticizing effect can be exhibited, which in turn reduces irritation to the skin.

By setting the content of the organic liquid component in the crosslinked adhesive layer (B) for generally not more than 200 parts by weight, preferably not more than 180 parts by weight, particularly preferably not more than 150 parts by weight, per 100 parts by weight of the adhesive in the crosslinked adhesive layer (B), reduction of cohesive power due to too much plasticizing of the adhesive layer can be prevented, which in turn solves the problem of increased irritation to the skin again due to adhesive residues upon peeling, even if the adhesive has been subjected to the crosslinking treatment.

While the substrate of the patch according to the present invention is not particularly limited, a laminate of a plastic film and a non-woven fabric, particularly a laminate film of a plastic film and a non-woven fabric is preferable.

The thickness of the substrate is generally 2-2000 µm, preferably 2-600 µm, particularly preferably 10-150 µm.

As the plastic film to be used for the laminate of a plastic film and a non-woven fabric, for example, films of polyester (e.g., PET (polyethylene terephthalate) and the like), ethylene/vinyl acetate copolymer, polyethylene, polyurethane, polyolefin, polypropylene and the like can be mentioned. Of these, a polyester film and a polyethylene film are preferable, and a polyester film is particularly preferable because a drug does not easily migrate into the substrate.

The thickness of the plastic film is generally 1-1000 µm, preferably 2-100 µm. From flexibility and handling, it is particularly preferably 5-50 µm.

The non-woven fabric to be used for the laminate of the plastic film and the non-woven fabric is not particularly limited, and can be produced from the materials generally used in the field of the patch. Examples of such material include polyester (e.g., PET (polyethylene terephthalate) and the like), polyethylene, polypropylene, polyamide and the like, with preference given to polyester, polypropylene and polyamide. The basis weight of the non-woven fabric is generally 1-100 g/m², preferably 6-50 g/m², particularly preferably 6-30 g/m², in view of fine flexibility and the fine feel of adhesion to the skin upon application.

The thickness of the non-woven fabric is generally 1-1000 µm, preferably 3-500 µm, particularly preferably 5-100 µm.

The patch of the present invention preferably comprises a substrate which is a laminate of a plastic film and a non-woven fabric as mentioned above, wherein the adhesive layer (A) is laminated on the non-woven fabric side. By laminating the adhesive layer (A) on the non-woven fabric layer of the substrate, the anchor force for the substrate can be increased, even when the adhesive to be used for the adhesive layer (A) is a non-crosslinked adhesive having a low cohesive power, and the like. Even when the adhesive in the adhesive layer (A) has a low cohesive power, a cohesive failure of the patch upon peeling off from the skin, which is due to insufficient cohesive power, can be prevented.

By laminating the crosslinked adhesive layer (B) on the adhesive layer (A), moreover, what is called an adhesive residue, wherein a part of the adhesive remains on the skin surface and the like upon peeling therefrom of the patch after application, and the like can be prevented, and what is called an adhesive bleed, wherein a part of the adhesive bleeds out inside the package during preservation and the resulting performance of taking out the patch from the package can be improved.

The thickness of the adhesive layer (A) varies depending on the kind of substrate, the adhesive to be used for the adhesive layer (A), and the like, but it is generally 5-200 µm, preferably 10-150 µm, particularly preferably 20-100 µm.

Herein, the thickness of the adhesive layer (A), when an adhesive solution is directly applied to one surface of a substrate (e.g., by comma direct, comma reverse, rip direct, rip reverse, gravure coating and the like) and dried, or what is called a direct coating, is generally the distance between the adhesive layer surface and the boundary of the substrate and the adhesive layer. When an adhesive layer is directly formed on the non-woven fabric surface of the substrate, which is a laminate of a non-woven fabric and a plastic film and the like, the adhesive layer may be embedded in the non-woven fabric, in other words, the adhesive layer is physically embedded in the non-woven fabric or the non-woven fabric is impregnated with the adhesive. In this case, the thickness of the adhesive layer (A) is the distance between the surface of the adhesive layer and the boundary of the non-woven fabric and the plastic film and the like. In the case of what is called a transfer coating, wherein an adhesive solution is applied onto a separator and dried to form an adhesive layer and the adhesive layer is then adhered to one surface of a substrate, the thickness of the adhesive layer (A) refers to the thickness of an adhesive layer formed by applying and drying on a separator.

In addition, when the adhesive layer (A) is formed on a non-woven fabric of a substrate consisting of a laminate of a plastic film and a non-woven fabric, the thickness of the adhesive layer (A) is preferably determined in consideration of the correlation to the thickness of the non-woven fabric of the substrate.

When the adhesive layer (A) is formed by what is called a direct coating, the adhesive layer (A) is preferably not completely embedded in the non-woven fabric, because when the adhesive layer (A) is completely embedded in the non-woven fabric, adhesion to the crosslinked adhesive layer (B) to be laminated further becomes insufficient, which in turn may result in insufficient migration of the drug into the skin surface during application, as well as adhesive residue due to interfacial peeling between the both adhesive layers upon peeling off of the patch after application.

In contrast, when the adhesive layer (A) outside the non-woven fabric (or adhesive not in contact with the non-woven fabric) is thick, a cohesive failure occurs in the adhesive outside the non-woven fabric, possibly leaving an adhesive residue when peeling the patch after adhesion and the like. Accordingly, the adhesive layer (A) is preferably almost embedded in the non-woven fabric of the substrate and extremely slightly outside the non-woven fabric. The thickness of the adhesive layer (A) outside the non-woven fabric is specifically 0-100 µm, preferably 0-50 µm, more preferably 0-10 µm.

Of the thickness of adhesive layer (A), the thickness ratio of the adhesive layer within the non-woven fabric layer: adhesive layer outside the non-woven fabric is generally 100:0 - 25:75, preferably 100:0 to 50:50, from the above-mentioned aspect.

In contrast, when the adhesive layer (A) has been formed by what is called a transfer coating, and when the adhesive layer (A) is in contact with only the surface of the non-woven fabric and is thick, the adhesive suffers from a cohesive failure, highly possibly leaving an adhesive residue upon peeling the patch after adhesion and the like. Thus, before adhesion of the crosslinked adhesive layer (B), the substrate with the adhesive layer (A) is preferably subjected to an adhesion treatment with a heat roll and the like, thereby sufficiently embedding the adhesive layer (A) in the non-woven fabric layer of the substrate, after which crosslinked adhesive layer (B) is adhered thereto.

While the thickness of the crosslinked adhesive layer (B) varies depending on the kind of the adhesive to be used for the adhesive layer (B), and the like, it is generally 5-200 µm, preferably 7-150 µm, particularly preferably 10-100 µm.

The adhesive layer (A) and the crosslinked adhesive layer (B) may respectively contain additives such as antioxidants, various pigments, various fillers, stabilizers, drug-dissolution aids, drug-dissolution suppressors and the like as necessary. In this case, the total amount of the additive is in the range of preferably about 2-50 parts by weight per 100 parts by weight of the adhesive.

The patch of the present invention can be produced by, for example, a production method comprising the following steps (1)-(3) in this order. That is, the patch can be produced by step (1):
dissolving a non-crosslinked adhesive and DMAEs in a non-ester organic solvent to give an adhesive solution,
step (2):
applying (e.g., by comma direct, comma reverse, rip direct, rip reverse, gravure coating and the like) the above-mentioned adhesive solution onto one surface of a substrate, and drying the adhesive solution to form an adhesive layer (A), or applying (e.g., by comma direct, comma reverse, rip direct, rip reverse, gravure coating and the like) the above-mentioned adhesive solution on a separator (e.g., polyester film that underwent release treatment, and the like), drying the adhesive solution to form an adhesive layer and transfer-coating the adhesive layer on one surface of a substrate to form an adhesive layer (A), and
step (3):
forming a crosslinked adhesive layer (B) free of DMAEs on the adhesive layer (A).

As the solvent to be used for forming adhesive layer (A), a non-ester organic solvent is preferable in view of the reactivity with DMAEs. As the non-ester organic solvent, for example, at least one kind selected from the group consisting of toluene, hexane, methanol, ethanol and propanol can be mentioned, with preference given to a mixture of toluene or hexane and at least one kind selected from lower alcohols such as methanol, ethanol, propanol and the like. While the mixing ratio of the mixture varies depending on the adhesive to be used, the weight ratio of toluene or hexane and the total amount of lower alcohol is, for example, 99:1-70:30, preferably 90:10-60:40, in view of the solubility of adhesive and the drug.

In step (3), the crosslinked adhesive layer (B) can be obtained by, for example, dissolving the above-mentioned adhesive and the crosslinking agent in a suitable solvent (e.g., ethyl acetate etc.), applying the obtained adhesive solution to a separator (e.g., release-treated polyester film and the like) and drying the solution. When preparing the crosslinked adhesive layer (B), it is essential that the mixture of the adhesive and the crosslinking agent should not contain DMAEs. Because the mixture of the adhesive and the crosslinking agent does not contain DMAEs, inhibition of crosslinking of the adhesive can be avoided, which crosslinking is caused by the contact of the crosslinking agent and DMAEs.

The patch of the present invention comprises the aforementioned substrate, aforementioned adhesive layer (A) laminated on one surface of the substrate and the aforementioned crosslinked adhesive layer (B) laminated on the adhesive layer (A). It is preferable to cover and protect the exposed surface of the crosslinked adhesive layer (B) until just before adhesion to the skin surface, with a release liner such as paper, plastic film and the like release-treated by the application of a silicone resin, a fluororesin and the like. When in use, it is released to expose the crosslinked adhesive layer (B) and the patch is adhered to the adhesion site to administer the drug.

The shape of the patch is not limited and includes, for example, tape, sheet and the like.

The dose of the drug in the patch of the present invention varies depending on the age, body weight and conditions of patients, and the like, and a patch containing 5-60 mg of DMAEs is generally preferably adhered to 5-100 cm² of the skin of an adult at a frequency of about 1-3 times per 3 days.

### Best Mode for Embodying the Invention

### Examples

The patch of the present invention is explained in more detail by referring to the following Examples and Test Examples. It is needless to say that the present invention can be variously modified within the scope that does not deviate from the technical idea of the present invention. In the following context, % means wt%.

### Example 1

crosslinked adhesive layer (B)
   adhesive 60%
   (2-ethylhexyl acrylate/acrylic acid copolymer)
   isopropyl myristate 40%
   isocyanate crosslinking agent 0.15% (relative to adhesive solid content)
   (CORONATE HL: NIPPON POLYURETHANE INDUSTRY CO., LTD.)
DMAE-containing non-crosslinked adhesive layer (A)
   adhesive 26.7%
   (2-ethylhexyl acrylate/acrylic acid copolymer)
   isopropyl myristate 40%
   DMAE 33.3%

To a solution of an acrylic adhesive (prepared by copolymerization of 2-ethylhexyl acrylate/acrylic acid = 95/5) in ethyl acetate were added isopropyl myristate in a proportion of 40% of the plaster weight and CORONATE HL in a proportion of 0.15% of an adhesive solid content, and the solution was applied to a release-treated polyester film, so that the thickness after drying became 10 µm, dried and subjected to an aging treatment at 70°C for 48 hr to give a crosslinked adhesive layer (B).

To a solution of an acrylic adhesive (prepared by copolymerization of 2-ethylhexyl acrylate/acrylic acid = 95/5) in a mixed solvent of toluene/methanol were added DMAE in a proportion of 33.3% and isopropyl myristate in a proportion of 40% of the plaster weight, and this adhesive solution was applied to a non-woven fabric surface of a substrate made of a 6 µm thick PET film and a PET non-woven fabric having a basis weight of 8 g/m², so that the thickness after drying became 30 µm, and dried to give a non-crosslinked adhesive layer (A).

The crosslinked adhesive layer (B) prepared as mentioned above was laminated on the surface of the non-crosslinked adhesive layer (A) to give a DMAE tape.

### Example 2

crosslinked adhesive layer (B)
   adhesive 60%
   (2-ethylhexyl acrylate/acrylic acid copolymer)
   isostearyl alcohol 40%
   metal salt (metal chelate) crosslinking agent 0.3%
   (relative to adhesive solid content)
   (ALCH: Kawaken Fine Chemicals Co., Ltd.)
DMAE-containing non-crosslinked adhesive layer (A)
   adhesive 26.7%
   (2-ethylhexyl acrylate/acrylic acid copolymer)
   isostearyl alcohol 40%
   DMAE 33.3%

To a solution of an acrylic adhesive (prepared by copolymerization of 2-ethylhexyl acrylate/acrylic acid = 95/5) in ethyl acetate were added isostearyl alcohol in a proportion of 40% of the plaster weight and ALCH in a proportion of 0.3% of an adhesive solid content, and the solution was applied to a release-treated polyester film, so that the thickness after drying became 10 µm, dried and subjected to an aging treatment at 70°C for 48 hr to give a crosslinked adhesive layer (B).

To a solution of an acrylic adhesive (prepared by copolymerization of 2-ethylhexyl acrylate/acrylic acid = 95/5) in a mixed solvent of toluene/methanol were added DMAE in a proportion of 33.3% and isostearyl alcohol in a proportion of 40% of the plaster weight, and this adhesive solution was applied to a release-treated polyester film, so that the thickness after drying became 30 µm and dried, and the adhesive layer was adhered to a non-woven fabric surface of a substrate, which is made of a 6 µm thick PET film and a polyamide non-woven fabric having a basis weight of 20 g/m² to give a non-crosslinked adhesive layer (A).

The polyester film on the non-crosslinked adhesive layer (A) was peeled off and the crosslinked adhesive layer (B) prepared as mentioned above was laminated on the plaster surface of the layer (A) to give a DMAE tape.

### Example 3

crosslinked adhesive layer (B)
   adhesive 70%
   (2-ethylhexyl acrylate/acrylic acid/vinylpyrrolidone copolymer)
   hexyl decanol 30%
   metal salt (metal chelate) crosslinking agent 0.3%
   (relative to adhesive solid content)
   (ALCH: Kawaken Fine Chemicals Co., Ltd.)
DMAE-containing non-crosslinked adhesive layer (A)
   adhesive 43.3%
   (2-ethylhexyl acrylate/acrylic acid/vinylpyrrolidone copolymer)
   hexyl decanol 30%
   DMAE 26.7%

To a solution of an acrylic adhesive (prepared by copolymerization of 2-ethylhexyl acrylate/acrylic, acid/vinylpyrrolidone = 75/3/22) in ethyl acetate were added hexyl decanol in a proportion of 30% of the plaster weight and ALCH in a proportion of 0.3% of an adhesive solid content, and the solution was applied to a release-treated polyester film, so that the thickness after drying became 10 µm, dried and subjected to an aging treatment at 70°C for 48 hr to give a crosslinked adhesive layer (B).

To a solution of an acrylic adhesive (prepared by copolymerization of 2-ethylhexyl acrylate/acrylic acid/vinylpyrrolidone = 75/3/22) in ethanol were added DMAE in a proportion of 26.7% and hexyl decanol in a proportion of 30% of the plaster weight, and this adhesive solution was applied to the surface of a non-woven fabric of a substrate made of a 6 µm thick PET film and a PET non-woven fabric having a basis weight of 8 g/m², so that the thickness after drying became 30 µm and dried to give a non-crosslinked adhesive layer (A).

The crosslinked adhesive layer (B) prepared as mentioned above was laminated on the plaster surface of the non-crosslinked adhesive layer (A) to give a DMAE tape.

### Example 4

crosslinked adhesive layer (B)
   adhesive 60%
   (2-ethylhexyl acrylate/acrylic acid copolymer)
   isopropyl myristate 40%
   isocyanate crosslinking agent 0.15% (relative to adhesive solid content)
   (CORONATE HL: NIPPON POLYURETHANE INDUSTRY CO., LTD.)
DMAE-containing non-crosslinked adhesive layer (A)
   adhesive 26.7%
   (2-ethylhexyl acrylate/acrylic acid copolymer)
   isostearyl alcohol 40%
   DMAE 33.3%

To a solution of an acrylic adhesive (prepared by copolymerization of 2-ethylhexyl acrylate/acrylic acid = 95/5) in ethyl acetate were added isopropyl myristate in a proportion of 40% of the plaster weight and CORONATE HL in a proportion of 0.15% of an adhesive solid content, and the solution was applied to a release-treated polyester film, so that the thickness after drying became 10 µm, dried and subjected to an aging treatment at 70°C for 48 hr to give a crosslinked adhesive layer (B).

To a solution of an acrylic adhesive (prepared by copolymerization of 2-ethylhexyl acrylate/acrylic acid = 95/5) in a mixed solvent of toluene/methanol were added DMAE in a proportion of 33.3% and isostearyl alcohol in a proportion of 40% of the plaster weight, and this adhesive solution was applied to the surface of a non-woven fabric of a substrate made of a 6 µm thick PET film and a PET non-woven fabric having a basis weight of 8 g/m², so that the thickness after drying became 30 µm and dried to give a non-crosslinked adhesive layer (A).

The crosslinked adhesive layer (B) prepared as mentioned above was laminated on the plaster surface of the non-crosslinked adhesive layer (A) to give a DMAE tape.

### Example 5

In the same manner as in Example 1 except that isopropyl myristate was not added to the non-crosslinked adhesive layer (A) and the crosslinked adhesive layer (B), a DMAE tape was prepared.

### Example 6

In the same manner as in Example 2 except that ethyl acetate was used as a solvent for the non-crosslinked adhesive layer (A), a DMAE tape was prepared.

### Example 7

In the same manner as in Example 3 except that the non-crosslinked adhesive layer (A) was applied to a surface of the PET film of the substrate, a DMAE tape was prepared.

### Example 8

crosslinked adhesive layer (B)
   adhesive 60%
   (2-ethylhexyl acrylate/acrylic acid copolymer)
   isopropyl myristate 40%
   isocyanate crosslinking agent 0.15% (relative to adhesive solid content)
   (CORONATE HL: NIPPON POLYURETHANE INDUSTRY CO., LTD.)
DMAE-containing non-crosslinked adhesive layer (A)
   adhesive 26.7%
   (polyisobutylene type)
   isopropyl myristate 40%
   DMAE 33.3%

To a solution of an acrylic adhesive (prepared by copolymerization of 2-ethylhexyl acrylate/acrylic acid = 95/5) in ethyl acetate were added isopropyl myristate in a proportion of 40% of the plaster weight and CORONATE HL in a proportion of 0.15% of an adhesive solid content, and the solution was applied to a release-treated polyester film, so that the thickness after drying became 10 µm, dried and subjected to an aging treatment at 70°C for 48 hr to give a crosslinked adhesive layer (B).

To a solution of a rubber adhesive containing polyisobutylene as a main component in hexane were added DMAE in a proportion of 33.3% and isopropyl myristate in a proportion of 40% of the plaster weight, and this adhesive solution was applied to the surface of a non-woven fabric of a substrate made of a 6 µm thick PET film and a PET non-woven fabric having a basis weight of 8 g/m², so that the thickness after drying became 30 µm and dried to give a non-crosslinked adhesive layer (A).

The crosslinked adhesive layer (B) prepared as mentioned above was laminated on the plaster surface of the non-crosslinked adhesive layer (A) to give a DMAE tape.

### Comparative Example 1

In the same manner as in Example 1 except that an isocyanate crosslinking agent was not added to the crosslinked adhesive layer (B), a DMAE tape was prepared.

### Comparative Example 2

DMAE-containing non-crosslinked adhesive layer
   adhesive 35%
   (2-ethylhexyl acrylate/acrylic acid copolymer)
   isopropyl myristate 40%
   DMAE 25%

To a solution of an acrylic adhesive (prepared by copolymerization of 2-ethylhexyl acrylate/acrylic acid = 95/5) in a mixed solvent of toluene/methanol were added DMAE in a proportion of 25% and isopropyl myristate in a proportion of 40% of the plaster weight, and this adhesive solution was applied to a non-woven fabric surface of a substrate made of a 6 µm thick PET film and a PET non-woven fabric having a basis weight of 8 g/m², so that the thickness after drying became 40 µm and dried to give a non-crosslinked adhesive layer.

### Comparative Example 3

DMAE-containing crosslinked adhesive layer
   adhesive 35%
   (2-ethylhexyl acrylate/acrylic acid copolymer)
   isopropyl myristate 40%
   DMAE 25%
   isocyanate crosslinking agent 0.15% (relative to adhesive solid content)
   (CORONATE HL: NIPPON POLYURETHANE INDUSTRY CO., LTD.)

To a solution of an acrylic adhesive (prepared by copolymerization of 2-ethylhexyl acrylate/acrylic acid = 95/5) in ethyl acetate were added DMAE in a proportion of 25% and isopropyl myristate in a proportion of 40% of the plaster weight, and CORONATE HL in a proportion of 0.15% of the adhesive solid content, and this adhesive solution was applied to the surface of a non-woven fabric of a substrate made of a 6 µm thick PET film and a PET non-woven fabric having a basis weight of 8 g/m², so that the thickness after drying became 40 µm, dried and subjected to an aging treatment at 70°C for 48 hr to give a DMAE-containing crosslinked adhesive layer.

### Test Example 1: permeability test

The samples of Examples 1-8 obtained above were punched out in 6 mmφ, each was adhered to the center of a shed snake skin (diameter 2 cm), set on a permeability tester (manufactured by VANGARD International Inc., catalog No. VFT02), and the skin permeability of DMAE into water on the receptor side was measured. The cumulative permeation amount in 24 hr is shown in Table 1.

### Test Example 2: adhesion test

The samples of Examples 1-8 and Comparative Examples 1-3 obtained above were punched out in 10cm², and each was adhered to the skin of the back of New Zealand white rabbits, which had been sheared and shaved. The samples were peeled off 24 hr later and the adhesive properties relative to during adhesion and after peeling were measured according to the following scores. The results are shown in Table 1.
During adhesion
⊙: Fine adhesiveness in the entirety without lifting or peeling.
○: Lifting or peeling was observed to some extent but without practical problem.
Δ: Considerable lifting or peeling was observed but without falling
×: Peeling in the area of 50% or above, or falling.
After peeling
⊙ Fine peeling without adhesive residue on the adhered area.
○: Adhesive residue observed to some extent on the periphery.
Δ: Considerable adhesive residue was observed.
×: Adhesive residue was observed in the entirety.

### Test Example 3: stability test

The samples of Examples 1-8 obtained above immediately after production were punched out in 10cm², extracted with methanol and a reactant with DMAE was confirmed. The results are shown in Table 1.

**Table 1**

| Samples | Permeability test (µg/cm²/24h) | Adhesion test | | Stability test |
|---|---|---|---|---|
| | | During adhesion | After peeling | |
| Example 1 | 679.93 | ⊙ | ⊙ | No reactant observed |
| Example 2 | 557.59 | ⊙ | ⊙ | No reactant observed |
| Example 3 | 533.76 | ⊙ | ⊙ | No reactant observed |
| Example 4 | 649.78 | ⊙ | ⊙ | No reactant observed |
| Example 5 | 54.46 | ⊙ | ⊙ | No reactant observed |
| Example 6 | 550.35 | ⊙ | ⊙ | Generation of acetyl form observed (2.8%) |
| Example 7 | 524.89 | ○ | ○ (partial anchor failure) | No reactant observed |
| Example 8 | 329.89 | ○ | ○ (partial interfacial peeling) | No reactant observed |
| Comparative Example 1 | - | ⊙ | × (cohesive failure) | - |
| Comparative Example 2 | - | ⊙ | × (cohesive failure) | - |
| Comparative Example 3 | - | ⊙ | × (cohesive failure) | - |

| | | | | |
|---|---|---|---|---|
| -: not measured | | | | |

### Industrial Applicability

According to the present invention, the percutaneous absorbability of DMAEs can be improved and a patch free of the problems such as adhesive residue and adhesive bleed can be provided.

This application is based on a patent application No. 2002-110609 filed in Japan.

## Claims

1. A patch comprising a substrate, a non-crosslinked adhesive layer (A) comprising 2-amino-1-(2',5'-dimethoxyphenyl)ethanol or a pharmacologically acceptable salt thereof, which is laminated on one surface of the substrate, and a crosslinked adhesive layer (B) laminated on the adhesive layer (A).

2. The patch of claim 1, wherein the crosslinked adhesive layer (B) is obtained by crosslinking an adhesive with at least one kind of crosslinking agent selected from the group consisting of an isocyanate crosslinking agent, a metal salt crosslinking agent and an epoxy crosslinking agent.

3. The patch of claim 1, wherein the adhesive layer (A) and/or the crosslinked adhesive layer (B) comprise(s) an acrylic adhesive.

4. The patch of claim 1, wherein the adhesive layer (A) and/or the crosslinked adhesive layer (B) comprise(s) a long chain fatty acid ester and/or a long chain aliphatic alcohol.

5. The patch of claim 4, which satisfies at least one of the following (i) and (ii):
(i) the total content of the long chain fatty acid ester and/or the long chain aliphatic alcohol in the adhesive layer (A) is 25-200 parts by weight per 100 parts by weight of the adhesive in the adhesive layer (A),
(ii) the total content of the long chain fatty acid ester and/or the long chain aliphatic alcohol in the crosslinked adhesive layer (B) is 25-200 parts by weight per 100 parts by weight of the adhesive in the crosslinked adhesive layer (B).

6. The patch of claim 4, wherein the long chain fatty acid ester is an ester of a fatty acid having 8 to 30 carbon atoms and an alcohol having 1 to 18 carbon atoms and the long chain aliphatic alcohol has 8 to 30 carbon atoms.

7. The patch of claim 1, wherein the content of 2-amino-1-(2',5'-dimethoxyphenyl)ethanol or a pharmacologically acceptable salt thereof in the adhesive layer (A) is 0.5-60 wt% of the total weight of the adhesive layer (A).

8. The patch of claim 1, wherein the substrate is a laminate of a plastic film and a non-woven fabric and the adhesive layer (A) is laminated on the non-woven fabric side.

9. The patch of claim 1, wherein the adhesive in the adhesive layer (A) and the adhesive in the crosslinked adhesive layer (B) have the same composition.

10. A production method of a patch, which comprises the steps of
(1) dissolving a non-crosslinked adhesive and 2-amino-1-(2',5'-dimethoxyphenyl)ethanol or a pharmacologically acceptable salt thereof in a non-ester organic solvent to give an adhesive solution,
(2) applying the adhesive solution onto one surface of a substrate, and drying the adhesive solution to form an adhesive layer (A), or applying the adhesive solution onto a separator, drying the adhesive solution to form an adhesive layer and transfer-coating the adhesive layer on one surface of a substrate to form an adhesive layer (A), and
(3) forming a crosslinked adhesive layer (B) free of 2-amino-1-(2',5'-dimethoxyphenyl)ethanol and a pharmacologically acceptable salt thereof on the adhesive layer (A), in this order.

11. The method of claim 10, wherein the crosslinked adhesive layer (B) is obtained by crosslinking an adhesive with at least one kind of crosslinking agent selected from the group consisting of an isocyanate crosslinking agent, a metal salt crosslinking agent and an epoxy crosslinking agent.

12. The method of claim 10, wherein the non-ester organic solvent is at least one kind selected from the group consisting of toluene, hexane, methanol, ethanol and propanol.

## Patentansprüche

1. Pflaster, umfassend ein Substrat, eine unvernetzte Klebstoffschicht (A), die 2-Amino-1-(2',5'-dimethoxyphenyl)ethanol oder ein pharmakologisch annehmbares Salz davon umfasst und auf eine Fläche des Substrats laminiert ist, und eine vernetzte Klebstoffschicht (B), die auf die Klebstoffschicht (A) laminiert ist.

2. Pflaster gemäß Anspruch 1, wobei die vernetzte Klebstoffschicht (B) erhalten wird, indem man einen Kleber mit wenigstens einer Art von Vernetzungsmittel vernetzt, das aus der Gruppe ausgewählt ist, die aus einem Isocyanat-Vernetzungsmittel, einem Metallsalz-Vernetzungsmittel und einem Epoxy-Vernetzungsmittel besteht.

3. Pflaster gemäß Anspruch 1, wobei die Klebstoffschicht (A) und/oder die vernetzte Klebstoffschicht (B) einen Acrylkleber umfasst.

4. Pflaster gemäß Anspruch 1, wobei die Klebstoffschicht (A) und/oder die vernetzte Klebstoffschicht (B) einen langkettigen Fettsäureester und/oder einen langkettigen aliphatischen Alkohol umfasst.

5. Pflaster gemäß Anspruch 4, das wenigstens einer der folgenden Bedingungen (i) und (ii) genügt:
(i) der Gesamtgehalt des langkettigen Fettsäureesters und/oder des langkettigen aliphatischen Alkohols in der Klebstoffschicht (A) beträgt 25-200 Gewichtsteile pro 100 Gewichtsteile des Klebers in der Klebstoffschicht (A);
(ii) der Gesamtgehalt des langkettigen Fettsäureesters und/oder des langkettigen aliphatischen Alkohols in der vernetzten Klebstoffschicht (B) beträgt 25-200 Gewichtsteile pro 100 Gewichtsteile des Klebers in der vernetzten Klebstoffschicht (B).

6. Pflaster gemäß Anspruch 4, wobei der langkettige Fettsäureester ein Ester einer Fettsäure mit 8 bis 30 Kohlenstoffatomen und eines Alkohols mit 1 bis 18 Kohlenstoffatomen ist und der langkettige aliphatische Alkohol 8 bis 30 Kohlenstoffatome aufweist.

7. Pflaster gemäß Anspruch 1, wobei der Gehalt an 2-Amino-1-(2',5'-dimethoxyphenyl)ethanol oder des pharmakologisch annehmbaren Salzes davon in der Klebstoffschicht (A) 0,5 bis 60 Gew.-% des Gesamtgewichts der Klebstoffschicht (A) beträgt.

8. Pflaster gemäß Anspruch 1, wobei das Substrat ein Laminat aus einer Kunststofffolie und einem Vliesstoff ist und die Klebstoffschicht (A) auf die Vliesstoffseite laminiert ist.

9. Pflaster gemäß Anspruch 1, wobei der Kleber in der Klebstoffschicht (A) und der Kleber in der vernetzten Klebstoffschicht (B) dieselbe Zusammensetzung haben.

10. Herstellungsverfahren für ein Pflaster, das die folgenden Schritte umfasst:
(1) Auflösen eines unvernetzten Klebers und von 2-Amino-1-(2',5'-dimethoxyphenyl)ethanol oder eines pharmakologisch annehmbaren Salzes davon in einem organischen Nichtester-Lösungsmittel unter Bildung einer Klebstofflösung;
(2) Auftragen der Klebstofflösung auf eine Fläche eines Substrats und Trocknen der Klebstofflösung unter Bildung einer Klebstoffschicht (A) oder Auftragen der Klebstofflösung auf eine Trennfolie, Trocknen der Klebstofflösung unter Bildung einer Klebstoffschicht und Übertragen der Klebstoffschicht durch Transferbeschichtung auf eine Fläche eines Substrats unter Bildung einer Klebstoffschicht (A); und
(3) Bilden einer vernetzten Klebstoffschicht (B), die frei von 2-Amino-1-(2',5'-dimethoxyphenyl)ethanol und einem pharmakologisch annehmbaren Salz davon ist, auf der Klebstoffschicht (A), in dieser Reihenfolge.

11. Verfahren gemäß Anspruch 10, wobei die vernetzte Klebstoffschicht (B) erhalten wird, indem man einen Kleber mit wenigstens einer Art von Vernetzungsmittel vernetzt, das aus der Gruppe ausgewählt ist, die aus einem Isocyanat-Vernetzungsmittel, einem Metallsalz-Vernetzungsmittel und einem Epoxy-Vernetzungsmittel besteht.

12. Verfahren gemäß Anspruch 10, wobei das organische Nichtester-Lösungsmittel wenigstens eine Art ist, die aus der Gruppe ausgewählt ist, die aus Toluol, Hexan, Methanol, Ethanol und Propanol besteht.

## Revendications

1. Timbre transdermique comprenant un substrat, une couche adhésive non réticulée (A) comprenant le 2-amino-1-(2',5'-diméthoxyphényl)éthanol ou un sel pharmacologiquement acceptable de celui-ci, qui est stratifiée sur une surface du substrat, et une couche adhésive réticulée (B) stratifiée sur la couche adhésive (A).

2. Timbre transdermique selon la revendication 1, dans lequel la couche adhésive réticulée (B) est obtenue par la réticulation d'un adhésif avec au moins un type d'agent de réticulation choisi dans le groupe constitué par un agent de réticulation de type isocyanate, un agent de réticulation de type sel métallique et un agent de réticulation de type époxy.

3. Timbre transdermique selon la revendication 1, dans lequel la couche adhésive (A) et/ou la couche adhésive réticulée (B) comprend/ comprennent un adhésif acrylique.

4. Timbre transdermique selon la revendication 1, dans lequel la couche adhésive (A) et/ou la couche adhésive réticulée (B) comprend/ comprennent un ester d'acide gras à longue chaîne et/ou un alcool aliphatique à longue chaîne.

5. Timbre transdermique selon la revendication 4, qui satisfait au moins un des points (i) et (ii) suivants :
(i) la teneur totale de l'ester d'acide gras à longue chaîne et/ou de l'alcool aliphatique à longue chaîne dans la couche adhésive (A) est de 25 à 200 parties en poids pour 100 parties en poids de l'adhésif dans la couche adhésive (A),
(ii) la teneur totale de l'ester d'acide gras à longue chaîne et/ou de l'alcool aliphatique à longue chaîne dans la couche adhésive réticulée (B) est de 25 à 200 parties en poids pour 100 parties en poids de l'adhésif dans la couche adhésive réticulée (B).

6. Timbre transdermique selon la revendication 4, dans lequel l'ester d'acide gras à longue chaîne est un ester d'un acide gras ayant de 8 à 30 atomes de carbone et un alcool ayant de 1 à 18 atomes de carbone et l'alcool aliphatique à longue chaîne possède de 8 à 30 atomes de carbone.

7. Timbre transdermique selon la revendication 1, dans lequel la teneur en 2-amino-1-(2'5'-diméthoxyphényl)éthanol ou un sel pharmaceutiquement acceptable de celui-ci dans la couche adhésive (A) est de 0,5 à 60 % en poids du poids total de la couche adhésive (A).

8. Timbre transdermique selon la revendication 1, dans lequel le substrat est un stratifié d'un film plastique et d'un tissu non tissé et la couche adhésive (A) est stratifiée sur le côté du tissu non tissé.

9. Timbre transdermique selon la revendication 1, dans lequel l'adhésif dans la couche adhésive (A) et l'adhésif dans la couche adhésive réticulée (B) ont la même composition.

10. Procédé de production d'un timbre transdermique, qui comprend les étapes de
(1) dissoudre un adhésif non réticulé et le 2-amino-1-(2',5'-diméthoxyphényl)éthanol ou un sel pharmacologiquement acceptable de celui-ci dans un solvant organique autre qu'un ester pour donner une solution d'adhésif,
(2) appliquer la solution d'adhésif sur une surface d'un substrat, et sécher la solution d'adhésif pour former une couche adhésive (A), ou appliquer la solution d'adhésif sur un séparateur, sécher la solution d'adhésif pour former une couche adhésive et enduire par transfert la couche adhésive sur une surface d'un substrat pour former une couche adhésive (A), et
(3) former une couche adhésive réticulée (B) exempte de 2-amino-1-(2',5'-diméthoxyphényl)-éthanol ou d'un sel pharmacologiquement acceptable de celui-ci sur la couche adhésive (A), dans cet ordre.

11. Procédé selon la revendication 10, dans lequel la couche adhésive réticulée (B) est obtenue par la réticulation d'un adhésif avec au moins un type d'agent de réticulation choisis dans le groupe constitué par un agent de réticulation de type isocyanate, un agent de réticulation de type sel métallique et un agent de réticulation de type époxy.

12. Procédé selon la revendication 10, dans lequel le solvant organique autre qu'un ester est au moins un type choisi dans le groupe constitué par le toluène, l'hexane, le méthanol, l'éthanol et le propanol.
